**Europäisches Patentamt**

(19) **European Patent Office**

**Office européen des brevets**

(11) Publication number : **0 340 919 B1**

# (12) EUROPEAN PATENT SPECIFICATION

(45) Date of publication of patent specification :
15.07.92 Bulletin 92/29

(51) Int. Cl.⁵ : **A61F 2/38**

(21) Application number : **89303454.6**

(22) Date of filing : **07.04.89**

(54) **A system of inserts for the tibial component of a knee prosthesis.**

(30) Priority : **08.04.88 US 179428**

(43) Date of publication of application :
**08.11.89 Bulletin 89/45**

(45) Publication of the grant of the patent :
**15.07.92 Bulletin 92/29**

(84) Designated Contracting States :
**BE CH DE FR GB IT LI LU NL SE**

(56) References cited :
**EP-A- 0 246 050**

(73) Proprietor : **Smith & Nephew Richards, Inc.**
**1450 Brooks Road**
**Memphis Tennessee 38116 (US)**

(72) Inventor : **Gustilo, Ramon B.**
**2545 Huntington Avenue South**
**Minneapolis Minnesota 55416 (US)**
Inventor : **Rand, James A.**
**1934 Westward Court Southwest**
**Rochester Minnesota 55902 (US)**
Inventor : **Brosnahan III, Robert E.**
**7057 Cross Timber Lane**
**Memphis Tennessee 38125 (US)**
Inventor : **Buford III, Thomas B.**
**3597 Hyacinth**
**Memphis Tennessee 38115 (US)**
Inventor : **Lackey, Jennifer J.**
**79 East Lafayette Circle**
**Memphis Tennessee 38111 (US)**
Inventor : **Roberts, Jeffrey G.**
**6248 Rockledge Drive**
**Bartlett Tennessee 38134 (US)**

(74) Representative : **Harrison, Philippa Dinah et al**
**A. A. THORNTON & CO Northumberland**
**House 303-306 High Holborn**
**London WC1V 7LE (GB)**

## Description

## BACKGROUND OF THE INVENTION

The present invention relates to knee prostheses. More particularly, the invention relates to a replacement system of the tibial component of a knee prosthesis which includes the use of inserts adapted to engage the undersurface of the tibial portion of the knee prosthesis to accommodate bony defects on the upper portion of the tibia.

In the total replacement of a knee joint, a knee prosthesis includes an upper femoral component which is a component secured to the upper femoral condyles that have been prepared and sculptured to secure the component in place. The second portion of the prosthetic knee includes a lower tibial component, which in a manner similar to that of the femoral component, is secured to the upper surface of a tibia that has been prepared to receive the tibial component. The third portion of the prothesis is a patella member which would be of common construction. The tibial component typically includes an upper platform or portion having a pair of concavities shaped to receive in nested relation the curved surfaces of a pair of condyle shaped segments formed on the femoral component in order to provide an articulation surface. These components are positioned and shaped to duplicate as closely as possible the natural bending movement of the knee. The lower portion of the tibial component is necessarily secured to the upper portion of the prepared tibia so as to provide a firm base of contact between the tibial component and the tibia.

In securing the tibial component to the upper portion of the tibia it is important that the overall condition of the hard outer bone layer of the tibia is able to support the weight of the body. In the course of surgery, as the tibia is being prepared to receive the tibial component, the tibia is typically sculptured to include a flat upper face so that a lower surface of the tibial component can rest on the tibia face with optional further attachment through a post member that fits into the medullary canal of the tibia. Bone cement may be used to secure the post to the tibia.

One problem that confronts surgeons during this type of procedure, is that oftentimes the outer surface of the hard bone on the tibia has eroded away. The extent of this may not be fully realized by X-rays or pre-surgery examination and thus the surgeon is not aware of the extent of the problem until surgery is underway. At this time, the surgeon must make critical decisions during a limited period of time and affix the tibial component to the prepared tibial portion. However, if the upper outer or inner portion of the tibia is soft or has eroded, proper spatial placement of the tibial component would cause a space to develop between the lower surface of the component and the upper surface of the tibia.

In order to correct this situation during the limited time available, surgeons have in the past improvised by positioning a sliver of bone or a bone graft or a portion of cement in order to fill the gap between the hard surface of the tibia and the undersurface of the tibial component. Although both of these alternatives provide some support to the tibial component, neither of them operates to firmly secure the tibial component in place. Another alternative is to resect additional bone to a level below the bony defect upon which the component can be mounted. This procedure is not preferred because in some cases a shortening of the tibia to a certain degree results in a corresponding shortening of that particular leg of the patient.

Therefore, a need has arisen for an acceptable insert positioned between the upper surface of the tibia that has been eroded away and the tibial component to avoid having to remove additional bone from the tibia, and to maintain the tibial component in the same anatomical position for maximum registration with the femoral component after the knee joint has been reconstructed.

According to a first aspect of the present invention there is provided a spacer system comprising a component of a bone prosthesis including an inferior face adapted to engage adjacent bone; and a spacer for positioning between the inferior face of the component and the adjacent bone, said spacer including a first surface for engaging the inferior face of the component and a second surface for engaging the adjacent bone, the first surface of the spacer and the inferior face of the component being provided with a plurality of projections and projection receiving portions sized and shaped to co-operably register with each other in a plurality of different relative positions and to maintain registration between the spacer and the component in a chosen one of said relative positions.

The projection receiving portions advantageously comprise one or more channels provided in one of the first surface of the spacer and the inferior face of the component and the projections on the other of said first surface and inferior face, the projections being aligned to register in said one or more channels. The one or more channels are preferably provided in the inferior face of the component and said projections on the first surface of the spacer. Both the first and second surfaces of the spacer may each be provided with a plurality of projections.

The inferior face of the component may include opposing hemispheres and the first and second surfaces of the spacer may have a similar array of projections so that the spacer may be positioned in either hemisphere of the component to allow interchangeability of the spacer between the two hemispheres.

The component may include a superior face adapted to receive a tibial platform thereupon. Furthermore, the component may have a curved outer edge and the spacer include an edge portion configured to align with a portion of the outer edge of the component.

The component may further include a post member extending inferiorly of said inferior face to provide support into the medullary canal of the bone. The spacer may advantageously be wedge-shaped in cross-section, with its first and second surfaces being convergent with respect to each other.

The component may have a curved outer edge and the first and second surfaces of the spacer may be configured to a generally oblong shape and provide a curved edge portion alignable with a portion of the curved outer edge of the component.

Alternatively the component may have a curved outer edge and the first and second surfaces of the spacer may be configured to a generally triangular shape and provide a curved edge portion alignable with a portion of the curved outer edge of the component.

A yet further alternative is for the component to have a curved outer edge and the first and second surfaces of the spacer to be configured to a generally oval shape and provide a curved edge portion alignable with a portion of the curved outer edge of the component.

According to a further aspect of the present invention there is provided a tibial component of a knee prosthesis comprising a general base portion having an inferior face for positioning against the upper face of a tibial bone, a superior face and a generally curved outer edge, the inferior face including a plurality of substantially parallel channels therein; and an insert positionable between the upper face of the tibial bone and the inferior face of the base portion, the insert having an outer edge with a portion thereof curved and including a plurality of projections spaced apart to register with the channels in the inferior face in various positions along the inferior face, said various positions including a position of substantial alignment between portions of the curved outer edges of both the base portion and the insert.

According to a yet further aspect of the present invention there is provided a tibial component of a knee prosthesis comprising a base portion having an inferior face for positioning against the upper face of a tibial bone and a superior face, the inferior face being provided with a plurality of projection receiving portions; and an insert positionable between the upper face of the tibial bone and the inferior face of the base portion, the insert being provided with a plurality of projections spaced apart to register with the projection receiving portions in the inferior face in various positions along the inferior face, said various positions including a position of substantial alignment between portions of the outer edges of both the base portion and the insert.

For a further understanding of the nature of the present invention, reference should be had to the following detailed description, taken in conjunction with the accompanying drawings, in which like parts are given like reference numerals, and wherein:

FIGURES 1-4 are underside, plan, and side views respectively of the tibial base component of the apparatus of the present invention;

FIGURE 5 is a partial cross-sectional view of the tibial component mounted on a tibia in the preferred embodiment of the apparatus of the present invention;

FIGURES 6-13 are views of the plurality of types of inserts used in the system of the present invention; and

FIGURES 14-18 are various views of the inserts in various positions on the underside of the tibial component of the preferred embodiment of the present invention.

## DETAILED DESCRIPTION OF THE PREFERRED EMBODIMENT

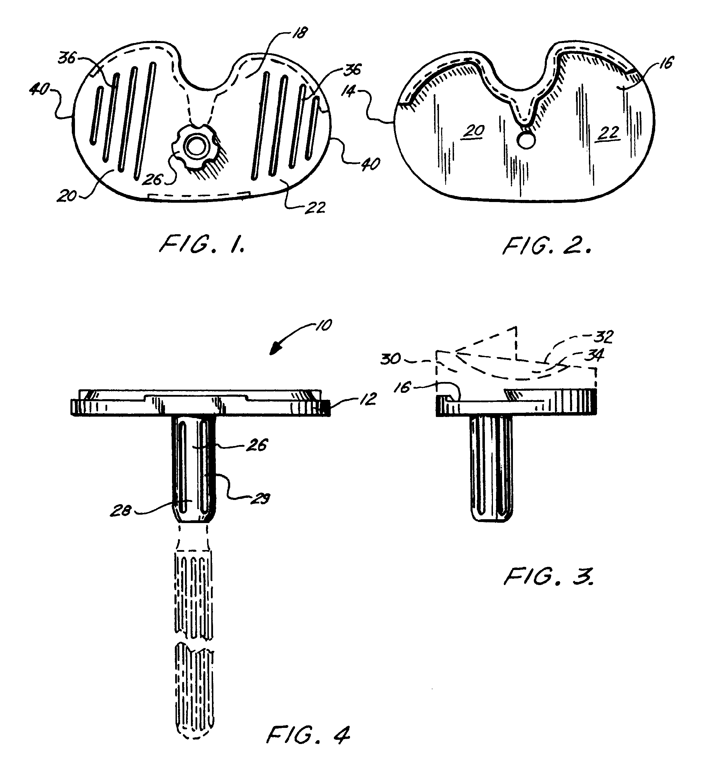

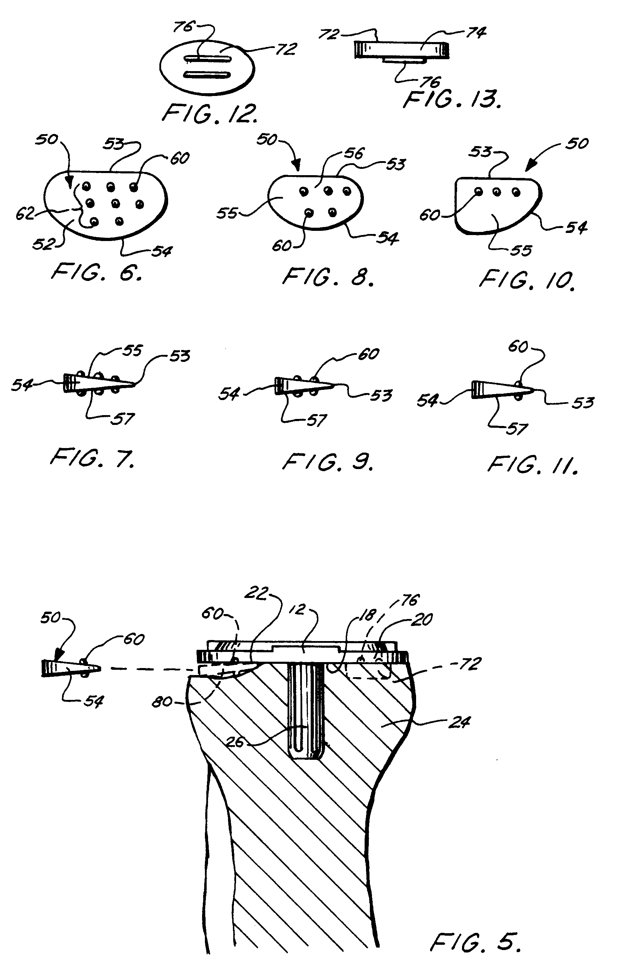

The preferred embodiment of the system of the present invention is illustrated in the Figures, with FIGURES 1 - 4 illustrating in particular the tibial component 10. As seen in the Figures, tibial component 10 includes a flat, assymetrically shaped base 12 having a superior flat face 16 and an inferior face surface 18, with base 12 substantially defining a left hemisphere 20, a right hemisphere 22, with the left hemisphere 20 and right hemisphere 22 substantially shaped to be received upon the pair of condyles of the lower tibia bone 24 as seen in FIGURE 5. The base 12 has attached thereto a lower mounting member or shaft 26 having a plurality of ridges 28 along the length of mounting member 26, securable into the interior top portion of tibia 24, as further illustrated FIGURE 5. In addition, as illustrated in FIGURE 4, mounting member 26 may be adapted with an extension frictionally engaged into a bore in member 26, to serve as an extension of member 26 for achieving a firmer mount between component base 12 and the condyles of tibia 24. For purposes of secure mounting, post 26 may be provided with cement in the grooves 29 defined between the ridges 28 so as to secure the base 12 in the inserted position.

In terms of function, in FIGURE 3 there is illustrated in phantom view an upper tibial portion 30 which is mountable on the superior or upper surface 16 of base member 12, the platform 30 defining an upper sloping surface 32 and having a pair of oblong concavities 34 (only one of which is shown) for receiving condyler portions of the upper femoral component of the total knee prosthesis. For purposes of this application, the upper platform 30 of the tibial component is standard, and will not be discussed further.

As seen in FIGURE 1, inferior or under surface 18 of tibial component base 12 is illustrated having post 26 extending inferiorly therefrom, and with left and right hemispheres 20 and 22 having a plurality of channels 36 in parallel relation, each of hemispheres 20 and 22 housing a series of four of said channels 36 with the length of the channels decreasing as the channels are positioned closer to the outer edge 40 of base portion 12. Therefore, when base portion 12 is positioned on the tibia as indicated in FIGURE 5, parallel channels 36 are positioned against the upper surface 25 of the tibia. The number of channels 36 may vary, and for purposes of explanation from channels 36 in each hemispheres 20 and 22 are noted.

Turning now to FIGURES 6 - 13, a plurality of wedges of inserts 50 are depicted. In the embodiment illustrated, there are four principal types of inserts 50 which would be utilized in the present system. FIGURE 6 illustrates in top view the largest of the inserts 52 which is generally oblong and has a first inner edge 53 and a continuous arcuate outer edge 54 which would conform substantially to the outer perimeter 40 of each hemisphere of tibial base 12 when the insert 52 is mounted in position intermediate the tibial bone 24 and the base 12 as seen in FIGURE 5. FIGURE 8 illustrates a smaller insert 56 which is substantially the same oblong shape of the larger insert 52 and thus has the same configured inner edge 53 and outer edge 54. FIGURE 10 illustrates a substantially triangular shaped insert 58 which has an inner flat edge 53 and an arcuate outer edge 54 for aligning with the outer edge of base 12 upon implantation.

The profile construction of the inserts are illustrated in the FIGURES 7, 9, and 11. Each insert is wedge-shaped, having the substantially straight edge 53 at the thinnest or shallowest point in the wedge with the arcuate edge 54 constituting the thickest part of the insert to form the wedge shape as illustrated in FIGURES 7, 9, and 11. This particular arrangement is substantially identical to each of the types of wedges as illustrated.

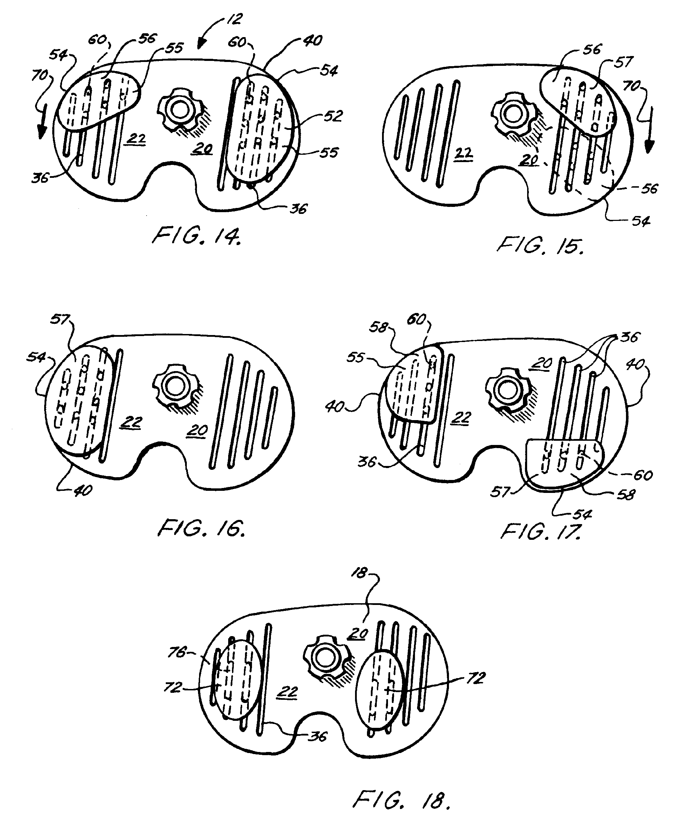

A unique feature of the wedges 50 is that each includes a plurality of projections 60 which are placed in a particular configuration upon both the superior surface 55 and inferior surface 57 of each insert so that as illustrated in FIGURE 6, the largest wedge 52 includes on each surface 55 and 57 three diagonal rows 62 across the width of the wedge. Each of the rows 62 is substantially in diagonal parallel relation, and is spaced apart from other rows so that each projection 60 would be positioned in a particular channel 36 as illustrated in FIGURES 14 - 17. Thus the rows 62 of projections on each insert extend generally from the inside edge 53 at an arcuate angle thereto to outside edge 54 and are positioned to align with the spaced channels 34 on the bottom face of the tibia base portion 12. The individual protrusions 60 are spaced equidistant from each other in each row 62 in order to form transverse rows approximately parallel to front edge 53.

Likewise, it should be noted in FIGURE 8 that the smaller wedge 56 has a plurality of projections 60, although the number of projections are reduced from the large wedge 52 due to the reduced surface area of the surfaces 55 or 57. Likewise as illustrated in FIGURE 10, triangular wedge 58 has a single row of projections 60 which would in effect be maintained in a single channel 36 along the inferior face 18 of base 12. The fourth principal wedge 72 of FIGURES 1213 will be described later.

The various positioning of the inserts 50 on the inferior face 18 of tibial base component 12 are illustrated in FIGURES 14 - 17. For example, in FIGURE 14, principal wedge 52 is positioned substantially in the hemisphere 20 with each of the three rows 62 of projections 60 aligned and registering in a channels 36, with the outer edge 54 in substantial alignment with the outer edge 40 of base member 12. Likewise, in that same Figure, smaller wedge 56 of FIGURE 8 - 9 includes projections 60 registering within channels 36. However, in this particular alignment, unlike the wedge 52, which could be considered a straight alignment along the length of channels 36, wedge 56 has been substantially rotated approximately 45° so that the edge 54 is aligned with the edge 40 of the hemisphere 22, and allows the positioning of this particular wedge along a different segment of the hemisphere, which may be required should that particular area of the bone be eroded or removal during surgery. It is noticed that the rows 62 such as in the principal insert of FIGURE 6 are spaced with the protrusions 60 an equidistant apart not only with a row 62 but also from a protrusion in an adjacent row to provide multiple positions of alignment with the grooves 36.

FIGURE 15 illustrates the use of the wedge 56 positioned in hemisphere 20 similar in position to wedge 56 as illustrated in FIGURE 14 as placed in hemisphere 22. This is accomplished by utilizing the series of projections 60 on the inferior face 57 of insert 56 when the insert is positioned in one hemisphere as compared to the other hemisphere. Therefore, there is a "mirror image" created in the utilization of the insert so that principal arcuate surface 54 is aligned with the outer edge 40 of hemisphere 20 in FIGURE 15. Although it is not illustrated, like large wedge 52, smaller wedge 56 could likewise be placed in straight alignments within channels 36 in addition to the position illustrated in FIGURE 15. Also, insert 56 can be slid or moved from the position as is illustrated in FIGURE 15 along the direction of Arrow 70, to a position (phantom view) wherein the lower portion of arcuate edge 54 would align with the lower curved edge of hemisphere 20.

FIGURE 16 illustrates large insert 52 having been moved from hemisphere 20 as shown in FIGURE 14 to hemisphere 22. Therefore, this maneuverability of the inserts along the length and across the channels allows the placement of one or more of the wedges 50 to cover almost any erosion that may occur in the particular bone area with just allowing a few wedges to be utilized.

As illustrated in FIGURE 17, generally triangular insert 58 of FIGURES 10 - 11, is utilized in hemisphere 22 in position with projections 60 registering with a single channel 36 with the arcuate edge 54 in alignment with the edge 40 of base 12. Also in that Figure, generally triangular wedge 58 of FIGURES 10 - 11 has been realigned in hemisphere 20 so that the projections 60 on inferior surface 57 have registered with three channels 36 and with the arcuate edge 54 of wedge 58 aligned with another point of the edge 40 of base 12 so that it may be utilized to cover a different area of an eroded bone.

In addition to the three principal wedges as discussed, there is included a fourth insert 72 illustrated FIGURES 12 and 13 and in FIGURE 18 mounted in position on the inferior surface 18 of base portion 12. As illustrated, insert 72 is is substantially oval shaped and has a uniform thickness along its edge 74. Rather than including a series of projections or protrusions 60, insert 72 includes a pair of raised, elongated projections or ridges 76 for registering with the pair of channels 36. For purposes of use, insert 72 would be utilized in substantially the central area of the tibia that may have eroded away, and is illustrated in phantom view in FIGURE 5 to compensate for a rather large and deep area of the pulp of the bone that may have eroded away with insert 72 set in position within the pulp so as to provide additional support.

For purposes of illustration, reference is made to FIGURE 5 where it is illustrated the base portion 12 of the tibial insert has been placed upon the upper surface of a tibia bone 24, with fixation post 26 inserted into the pulp of the bone. It is noted that a portion of the tibia 24 has eroded away or been removed substantially below hemisphere 22 of base portion 12, leaving an eroded space 80 between the upper portion of tibia bone 24 and the inferior surface 18 of component 12. Therefore, a typical insert 50, for example triangular insert 58 of FIGURES 10 - 11, would be placed into position between the upper portion of the eroded bone and the inferior surface 18 of component 12. The projections 60 are fitted into a single channel 36 in order to maintain insert 54 in position. In order to ensure further stability, insert 54 may be held in place via a bone cement which is common in the art. Therefore, once in place, insert 50 fills the void 80 created by the erosion or removal of bone, and therefore component 12 is maintained in fixed position atop tibia bone 24. Likewise, there is included in FIGURE 5 a phantom view of oval insert 72, which is shown position substantially below hemisphere 20 of component 12 to fill a void in the pulp area of bone 24 which may have been created by erosion of pulp in the core of the bone. Therefore, insert 72 would be placed in the eroded portion with its elongated projections or ridges 76 likewise registering with channels 36 in the inferior surface of face 18 of component 12.

For purposes of illustration, the particular series of inserts as illustrated in the Figures, is merely elucidative, and the shape of the various inserts may vary according to the need of the surgeon during surgery. However, it has been found that this series of inserts 52, 56, and 58 provides practically complete coverage of erosion that may occur along the edge of the tibia, and therefore provides the surgeon with a small yet substantially complete selection of inserts that he may utilize during surgery when a problem such as this would arise.

The illustrated embodiment thus provides a system of wedge-shaped spacer inserts which co-operate with the underside of the base of the tibial component and the upper portion of the tibia when the tibia has eroded away and a space has developed or when a space is created after the removal of undesirable soft bone. The system has a plurality of various shaped inserts, each insert having an upper and lower face, each face including a plurality of spaced, aligned protrusions adapted to engage a plurality of channels formed in the undersurface of the tibial base. The protrusions on the inserts register with the tibial base channels to allow positioning of the insert relative to the shape of the tibial component in various positions along the edge of the component so that the inserts are positioned to align themselves with the edge of the tibial component to provide a spacer between the bone and the undersurface of the component, which is secured within the same confined area as the component. In addition, due to the relative position of the projections on both faces of each insert, the same shaped insert may be utilized in either the left or right hemispheres of the tibial component. Therefore, the required configuration of inserts would be limited so that wedges in the left hemisphere would be moveable to the right hemisphere and therefore only require a minimum number of configured inserts for the surgeon to choose from during surgery.

The illustrated embodiment enables the surgeon to correct bone erosion or bone softness confronted during implantation of the tibial component, using a tibial component replacement system having a limited number of inserts which can be utilized in both hemispheres of the tibial component to replace bone that has eroded or is removed during surgery.

## Claims

1. A spacer system comprising :
a) a component (12) of a bone prosthesis including an inferior face (18) adapted to engage adjacent bone (24); and
b) a spacer (50,72) for positioning between the inferior face (18) of the component and the adjacent bone (24), said spacer including a first surface (55) for engaging the inferior face of the component and a second surface (57) for engaging the adjacent bone, the first surface of the spacer and the inferior face of the component being provided with a plurality of projections and projection receiving portions (36,60,76) sized and shaped to co-operably register with each other in a plurality of different relative positions and to maintain registration between the spacer (50,72) and the component (12) in a chosen one of said relative positions.

2. A spacer system as claimed in Claim 1, wherein the projection receiving portions (36) comprise one or more channels provided in one of the first surface of the spacer (55) and the inferior face (18) of the component.

3. A spacer system as claimed in Claim 2, wherein the projections (60,76) are provided on the other of said first surface (-55) and inferior face (18) and are aligned to register in said one or more channels (36).

4. A spacer system as claimed in Claim 3, wherein said one or more channels (36) are provided in the inferior face (18) of the component and said projections (60,76) on the first surface of the spacer.

5. A spacer system as claimed in any of the preceding claims, wherein both the first and second surfaces (55,57) of the spacer (50) are each provided with a plurality of projections (60).

6. A spacer system as claimed in any of the preceding claims, wherein the inferior face (18) of the component includes opposing hemispheres (20,22) and the first and second surfaces (55,57) of the spacer (50) have a similar array of projections (60) so that the spacer may be positioned in either hemisphere of the component to allow interchangeability of the spacer between the two hemispheres.

7. A spacer system as claimed in any of the preceding claims, wherein the component (12) includes a superior face (16) adapted to receive a tibial platform (30) thereupon.

8. A spacer system as claimed in any of the preceding claims, wherein the component (12) has a curved outer edge (40) and said spacer (50) includes an edge portion (54) configured to align with a portion of the outer edge of the component.

9. A spacer system as claimed in any of the preceding claims, wherein the component (12) further includes a post member (26) extending inferiorly of said inferior face (18) to provide support into the medullary canal of the bone (24).

10. A spacer system as claimed in any of the preceding claims, wherein the spacer (50) is wedge-shaped in cross-section.

11. A spacer system as claimed in any of the preceding claims, wherein the first and second surfaces (55,57) are convergent with respect to each other.

12. A spacer system as claimed in any of the preceding claims, wherein the component (12) has a curved outer edge and the first and second surfaces (55,57) of the spacer (50) are configured to a generally oblong shape and provide a curved edge portion (54) alignable with a portion (40) of the curved outer edge of the component.

13. A spacer system as claimed in any of Claims 1 to 11, wherein the component (12) has a curved outer edge and the first and second surfaces (55,57) of the spacer are configured to a generally triangular shape and provide a curved edge portion (54) alignable with a portion (40) of the curved outer edge of the component.

14. A spacer system as claimed in any of Claims 1 to 11, wherein the component (12) has a curved outer edge and the first and second surfaces (55,57) of the spacer are configured to a generally oval shape and provide a curved edge portion (54) alignable with a portion (60) of the curved outer edge of the component.

15. A tibial component of a knee prosthesis comprising:
   a) a general base portion (12) having an inferior face (18) for positioning against the upper face of a tibial bone (24), a superior face (16) and a generally curved outer edge (40), the inferior face including a plurality of substantially parallel channels (36) therein; and
   b) an insert (50) positionable between the upper face of the tibial bone (24) and the inferior face (18) of the base portion (12), the insert having an outer edge (54) with a portion thereof curved and including a plurality of projections (60) spaced apart to register with the channels (36) in the inferior face (18) in various positions along the inferior face, said various positions including a position of substantial alignment between portions of the curved outer edges (40,54) of both the base portion (12) and of the insert (50).

16. A tibial component of a knee prosthesis comprising:
   a) a base portion (12) having an inferior face (18) for positioning against the upper face of a tibial bone (24) and a superior face (16), the inferior face being provided with a plurality of projection receiving portions (36); and
   b) an insert (50) positionable between the upper face of the tibial bone (24) and the inferior face (18) of the base portion (12), the insert being provided with a plurality of projections (60) spaced apart to register with the projection receiving portions (36) in the inferior face (18) in various positions along the inferior face, said various positions including a position of substantial alignment between portions of the outer edges (40,54) of both the base portion (12) and of the insert (50).

**Patentansprüche**

1. Abstandssystem mit:
a) einer komponenten (12) einer knochenprothese mit einer unteren Fläche (18), die geeignet ist, sich mit dem benachbarten knochen (24) zu verbinden; und
b) einem Abstandsstück (50, 72) zum Anordnen zwischen der unteren Fläche (18) der komponente und dem benachbarten Knochen, wobei das Abstandsstück eine erste Oberfläche (55) zum Verbindung mit der unteren Fläche der komponenten und eine zweite Oberfläche (57) zum Verbindung mit dem benachbarten knochen umfaßt, wobei die erste Oberfläche des Abstandsstücks und die innere Fläche der komponente mit einer Mehrzahl von Ausstülpungen und Ausstülpungen aufnehmenden Bereichen (36, 60, 76) versehen sind, die von einer derartigen Größe und Form sind, daß sie in einer Mehrzahl verschiedener relativer Stellungen zusammenwirkend zusammenpassen und die Paßform zwischen dem Abstandsstück (50, 72) und der komponenten (12) in einer ausgewählten der relativen Stellungen zu halten.

2. Abstandssystem nach Anspruch 1, wobei die die Ausstülpungen aufnehmenden Bereiche (36) einen oder mehrere Kanäle umfassen, die in einer der ersten Oberfläche des Abstandsstücks (55) und der unteren Fläche (18) der komponente vorgesehen sind.

3. Abstandssystem nach Anspruch 2, wobei die Ausstülpungen (60, 76) in der anderen der ersten Oberfläche des Abstandsstücks (55) und der unteren Fläche (18) der komponente vorgesehen sind.

4. Abstandssystem nach Anspruch 3, wobei der eine oder die mehreren Kanäle (36) in der unteren Fläche (18) der komponente und die Ausstülpungen (60, 76) auf der ersten Oberfläche des Abstandsstücks vorgesehen sind.

5. Abstandssystem nach einem der vorhergehenden Ansprüche, wobei sowohl die erste als auch die zweite Oberfläche (55, 57) des Abstandsstücks jeweils mit einer Mehrzahl von Ausstülpungen (60) versehen sind.

6. Abstandssystem nach einem der vorhergehenden Ansprüche, wobei die untere Fläche (18) der komponente sich gegenüber liegende Halbkugeln (20, 22) umfaßt und die erste und die zweite Oberfläche (55, 57) des Abstandsstücks (50) eine ähnliche Anordnung von Ausstülpungen (60) besitzen, so daß das Abstandsstück in jeder der Halbkugeln der komponente angeordnet werden kann, um eine Austauschbarkeit des Abstandsstücks zwischen den beiden Halbkugeln zu ermöglichen.

7. Abstandssystem nach einem der vorhergehenden Ansprüche, wobei die komponente (12) eine obere Fläche (16) umfaßt, die geeignet ist eine Tibialfläche (307 darauf aufzunehmen.

8. Abstandssystem nach einem der vorhergehenden Ansprüche, wobei die komponente (12) eine gekrümmte äußere kante (40) besitzt und das Abstandsstück (50) einen kantenbereich (54) besitzt, der so konfiguriert ist, daß er mit einem Bereich der äußeren kante der komponente ausgerichtet ist.

9. Abstandssystem nach einem der vorhergehenden Ansprüche, wobei die komponente (12) ein Pfostenelement (26) umfaßt, daß sich unter der unteren Fläche (18) erstreckt, um eine Stütze in dem Markkanal des knochens (24) zu bilden. 10, Abstandssystem nach einem der vorhergehenden Ansprüche, wobei das Abstandsstück im Querschnitt keilförmig ist.

11. Abstandssystem nach einem der vorhergehenden Ansprüche, wobei die erste und die zweite Oberfläche (55, 57) im Bezug zueinander konvergent sind.

12. Abstandssystem nach einem der vorhergehenden Ansprüche, wobei die komponente (12) eine gekrümmte Außenkante besitzt, und die erste und die zweite Oberfläche (55, 57) des Abstandsstücks (60) in einer allgemein länglichen Form ausgeführt sind und einen gekrümmten kantenbereich (54) bilden, der mit einem Bereich (40) der gekrümmten Äußeren kante der komponente ausrichtbar ist.

13. Abstandssystem nach einem der vorhergehenden Ansprüche, wobei die Komponente (12) eine gekrümmte Außenkante besitzt, und die erste und die zweite Oberfläche (55, 57) des Abstandsstücks (60) in einer allgemein dreieckigen Form ausgeführt sind und einen gekrümmten kantenbereich (54) bilden, der mit einem Bereich (40) der gekrümmten Äußeren Kante der komponente ausrichtbar ist.

14. Abstandssystem nach einem der vorhergehenden Ansprüche, wobei die komponente (12) eine gekrümmte Außenkante besitzt, und die erste und die zweite Oberfläche (55, 57) des Abstandsstücks (60) in einer allgemein ovalen Form ausgeführt sind und einen gekrümmten kantenbereich (54) bilden, der mit einem Bereich (60) der gekrümmten äußeren Kante der komponente ausrichtbar ist.

15. Tibialkomponente einer Knieprothese mit:
a) einem allgemeinen Basisbereich (12) mit einer unteren Fläche (18) zum Anordnen gegen die obere Fläche eines Tibialknochens (24), einer oberen Fläche (16) und einer allgemein gekrümmten äußeren kante (40), wobei die untere Fläche eine Mehrzahl von im wesentlichen parallelen Kanälen (36) darin umfaßt; und
b) einem zwischen der oberen Fläche des Tibialknochens (24) und der unteren Fläche (18) des Basisbereichs (12) positionierbaren Einsatz (50), wobei der Einsatz eine äußere Kante (54) besitzt, von der ein Bereich gekrümmt ist und eine Mehrzahl von Ausstülpungen (60) umfaßt, die voneinander getrennt sind, um mit den Kanälen (36) der unteren Fläche (18) in verschiedenen Stellungen entlang der unteren Fläche zusammenzupassen, wobei die verschiedenen Stellungen eine Stellung umfaßt, in der die Bereiche der gekrümmten äußeren kanten (40, 54) sowohl des Basisbereichs (12) als auch des Einsatzes (50) untereinander ausgerichtet sind.

16. Tibialkomponente einer Knieprothese mit:

a) einem allgemeinen Basisbereich (12) mit einer unteren Fläche (18) zum Anordnen gegen die obere Fläche eines Tibialknochens (24), und einer oberen Fläche (16), wobei die untere Fläche mit einer Mehrzahl von Ausstülpungen aufnehmenden Bereichen versehen ist; und

b) einem zwischen der oberen Fläche des Tibialknochens (24) und der unteren Fläche (18) des Basisbereichs (12) positionierbaren Einsatz (50), wobei der Einsatz eine Mehrzahl von voneinander getrennten Ausstülpungen (60) umfaßt, um mit den Ausstülpungen aufnehmenden Bereichen (36) in der unteren Fläche (18) in verschiedenen Stellungen entlang der unteren Fläche zusammenzupassen, wobei die verschiedenen Stellungen eine Stellung umfaßt, in der die Bereiche der gekrümmten äußeren kanten (40, 54) sowohl des Basisbereichs (12) als auch des Einsatzes (50) untereinander ausgerichtet sind.

## Revendications

1. Système de cales comprenant :

a) un composant (12) de prothèse osseuse présentant une -face inférieure (18) adaptée pour s'appuyer sur un os (24) adjacent ; et

b) une cale (50, 72) destinée à être positionnée entre la face inférieure (18) du composant et l'os (24) adjacent, ladite cale présentant une première surface (55) destinée à s'appuyer contre la face inférieure du composant et une deuxième surface (57) destinée à s'appuyer contre l'os adjacent, la première surface de la cale et la face inférieure du composant étant munies d'une pluralité de saillies et de portions (36, 60, 76) de réception des saillies, gui sont dimensionnées et conformées de manière à se placer en coïncidence en coopération entre elles dans une pluralité de positions relatives différentes et pour maintenir la coïncidence entre la cale (50, 72) et le composant (12) dans l'une choisie desdites positions relatives.

2. Système de cales selon la revendication 1, dans lequel les portions (36) de réception des saillies comprennent une ou plusieurs rainures prévues dans l'une des surfaces constituées par la première surface (55) de la cale et la face inférieure (18) du composant.

3. Système de cales selon la revendication 1, dans lequel les saillies (60, 76) sont prévues sur l'autre desdites surfaces, la première surface (55) ou la face inférieure (18), et sont alignées pour se placer en coïncidence avec ladite rainure (36) ou -lesdites rainures (36).

4. Système de cales selon la revendication 1, dans lequel ladite rainure (36) ou lesdites rainures (36) sont prévues dans la face inférieure (18) du composant et lesdites saillies (60, 76) sur la première surface de la cale.

5. Système de cales selon une quelconque des revendications précédentes, dans lequel les première et deuxième surfaces (55, 57) de la cale (50) sont munies chacune d'une pluralité de saillies (60).

6. Système de cales selon une quelconque des revendications précédentes, dans lequel la face inférieure (18) du composant comprend des hémisphères opposés (20, 22) et les première et deuxième surfaces (55, 57) de la cale (50) portent des dessins de saillies (60) analogues, de sorte que la cale peut être positionnée dans l'un ou l'autre des hémisphères du composant, ce qui assure l'interchangeabilité de la cale entre les deux hémisphères.

7. Système de cales selon une quelconque des revendications précédentes, dans lequel le composant (10) comprend une face supérieure (16) adaptée pour recevoir une plate-forme tibiale (30).

8. Système de cales selon une quelconque des revendications précédentes, dans lequel le composant (12) présente un bord extérieur courbe (40) et ladite cale (50) comprend une portion de bord (54) configurée pour s'aligner sur une portion du bord extérieur du composant.

9. Système de cales selon une quelconque des revendications précédentes, dans lequel le composant (12) comprend en outre une tige (26) qui s'étend vers le bas sous ladite face inférieure (18) pour prendre appui dans le canal médullaire de l'os (24).

10. Système de cales selon une quelconque des revendications précédentes, dans lequel la cale (50) est en forme de coin en section transversale.

11. Système de cales selon une quelconque des revendications précédentes, dans lequel les première et deuxième surfaces (55, 57) sont convergentes l'une par rapport à l'autre.

12. Système de cales selon une quelconque des revendications précédentes, dans lequel le composant (12) possède un bord extérieur courbe et les première et deuxième surfaces (55, 57) de la cale (50) sont configurées selon une forme sensiblement oblongue et présentent une portion de bord courbe (54) qui peut être alignée sur une portion (40) du bord extérieur courbe du composant.

13. Système de cales selon une quelconque des revendications 1 à 11, dans lequel le composant (12) présente un bord extérieur courbe et les première et deuxième surfaces (55, 57) de la cale sont configurées selon une forme sensiblement triangulaire et présentent une portion de bord courbe (54) qui peut être alignée sur une portion (40) du bord extérieur courbe du composant.

14. Système de cales selon une quelconque des revendications 1 à 11, dans lequel le composant (12) présente un bord extérieur courbe et les première et deuxième surfaces (55, 57) de la cale sont configurées selon une forme sensiblement ovale et présentent une portion de bord courbe (54) qui peut être alignée sur une portion (60) du bord extérieur courbe du composant.

15. Composant tibial d'une prothèse de genou, comprenant :

a) une portion de base générale (12) ayant une face inférieure (18) destinée à être positionnée contre la face supérieure d'un os (24) de tibia, une face supérieure (16) et un bord extérieur (40) sensiblement courbe, la face inférieure présentant une pluralité de rainures (36) sensiblement parallèles, et

b) un insert (50) qui peut être positionné entre la face supérieure de l'os (24) de tibia et la face inférieure (18) de la portion de base (12), l'insert présentant un bord extérieur (54) dont une portion est courbe, et comprenant une pluralité de saillies (60) espacées pour se mettre en coïncidence avec les rainures (36) de la face inférieure (18) dans différentes positions le long de la face inférieure, lesdites différentes positions comprenant une position d'alignement sensiblement exact entre les portions des bords extérieurs courbes (40, 54) de la portion de base (12) et de l'insert (50).

16. Composant tibial d'une prothèse de genou comprenant :

a) une portion de base (12) ayant une face inférieure (18) destinée à être positionnée contre la face supérieure de l'os (24) du tibia et une face supérieure (16), la face inférieure étant munie d'une pluralité de portions (36) destinées à recevoir des saillies ; et

b) un insert (50) pouvant être positionné entre la face supérieure de l'os (24) du tibia et la face inférieure (18) de la portion de base (12), l'insert étant muni d'une pluralité de saillies (60) espacées pour se mettre en coïncidence avec les portions (36) de réception des saillies de la face inférieure (18) dans différentes positions le long de la face inférieure, lesdites différentes positions comprenant une position d'alignement sensiblement exact entre des portions des bords extérieurs (40, 54) de la portion de base (12) et de l'insert (50).

FIG. 1.

FIG. 2.

FIG. 3.

FIG. 4

FIG. 12.

FIG. 13.

FIG. 6.

FIG. 8.

FIG. 10.

FIG. 7.

FIG. 9.

FIG. II.

FIG. 5.

FIG. 14.

FIG. 15.

FIG. 16.

FIG. 17.

FIG. 18.